# EUROPEAN PATENT SPECIFICATION

(11) **EP 0 541 267 B1**
(45) Date of publication and mention of the grant of the patent: **01.07.1998**
(21) Application number: 92309549.1
(22) Date of filing: 20.10.1992
(51) Int. Cl.: C07C 43/04, C07C 41/09, B01J 31/10

(54) **Synthesis of alkyl tert-alkyl ether using fluorocarbon sulfonic acid polymers on inert supports**
Herstellung von Alkyl-Tert-Alkylethern unter Verwendung von Fluorkohlenstoffsulfonsäurepolymeren auf inerten Trägern
Préparation d'éthers alkyls tertio-alkyls utilisant des polymères d'acides sulfoniques perfluorocarbones sur des supports inertes

(30) Priority: 25.10.1991 US 783015
(43) Date of publication of application: 12.05.1993
(73) Proprietor: Huntsman Specialty Chemicals Corporation, Austin, Texas 78761 (US)
(72) Inventor: Knifton, John Frederick, Austin, Texas 78750 (US)
(74) Representative: Goddar, Heinz J., Dr.

(56) References cited:
- EP-A- 0 235 998
- EP-A- 0 451 394
- GB-A- 2 082 177
- US-A- 4 822 921
- US-A- 4 827 048
- US-A- 4 918 244
- US-A- 5 081 318

## Description

This invention concerns an improved process for preparing alkyl tertiary-alkyl ethers such as methyl tertiary-butyl ether (MTBE) by the reaction of tertiary alcohol such as tertiary butanol and primary alcohol such as methanol in the presence of a catalyst comprising a fluorocarbon sulfonic acid polymer on an inert support. The invention is particularly advantageous in that, in the synthesis of MTBE for example, the reaction takes place in one-step, the catalyst exhibits total MTBE plus isobutylene selectivity close to 100% and levels of tert-butanol conversion as high as 86%-88% are achieved.

It is known to those skilled in the art that ethers, including unsymmetrical ethers, may be prepared by reacting an alcohol with another alcohol to form the desired product. The reaction mixture, containing catalyst and/or condensing agent may be separated and further treated to permit attainment of the desired product. Such further treatment commonly includes one or more distillation operations.

Methyl tert-butyl ether is finding increasing use as a blending component in high octane gasoline as the current gasoline additives based on lead and manganese are phased out. Currently all commercial processes for the manufacture of methyl tert-butyl ether are based upon the liquid-phase reaction of isobutylene and methanol (Eq. 1), catalyzed by a cationic ion-exchange resin (see, for example: Hydrocarbon Processing, Oct. 1984, p. 63; Oil and Gas J., Jan. 1, 1979, p. 76; Chem. Economics Handbook-SRI, Sept. 1986, p. 543-7051P). The cationic ion-exchange resins used in MTBE synthesis normally have the sulfonic acid functionality (see: J. Tejero, J. Mol. Catal., 42 (1987) 257; C. Subramamam et al., Can. J. Chem. Eng., 65 (1987) 613).

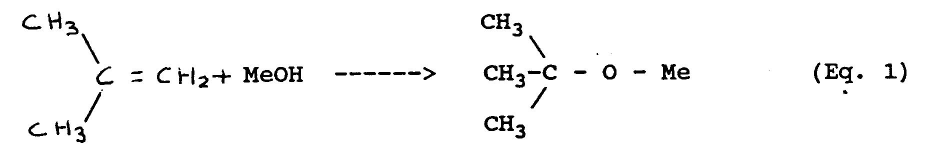

With the expanding use of MTBE as an acceptable gasoline additive, a growing problem is the availability of raw materials. Historically, the critical raw material is isobutylene (Oil and Gas J., June 8, 1987, p. 55). It would be advantageous, therefore, to have a process to make MTBE that does not require isobutylene as a building block. It would be advantageous to have an efficient process for making MTBE by reaction of methanol with tertiary butyl alcohol, since t-butanol (TBA) is readily available commercially through isobutane oxidation.

In US-A-4,144,138 (1979) to Rao et al., there is disclosed a method for recovering methyl tertiary butyl ether from etherification reaction effluent by azeotropic distillation to recover methanol-ether azeotrope overhead which is water-washed to give pure ether raffinate plus ether-methanol bottoms, the latter being azeotropically distilled to yield ether-methanol overhead which is recycled to water washing.

The preparation of methyl tert-butyl ether from methyl and tert-butyl alcohols is discussed in S. V. Rozhkov et al., Prevrashch Uglevodorodov, Kislotno-Osnovn. Geterogennykh Katal. Tezisy Dokl., Vses. Konf., 1977, 150 (C. A. 92:58165y). Here the TBA and methanol undergo etherification over KU-2 strongly acidic sulfopolystyrene cation-exchangers under mild conditions. This reference contains data on basic parameters of such a process. It is also pointed out that, although a plant for etherification over cation exchangers does not present any major problems, considerations include the fact that recycling large amounts of tert-butyl alcohol and methanol, as well as isobutylene, causes the scheme to be somewhat more expensive. Also, the progress of the reaction over cation exchangers is usually complicated by various adsorption and diffusion factors, by swelling phenomena, and by the variable distribution of the components between the solution and ion-exchanger phase. Furthermore, said acidic cation-exchangers with an organic (polystyrene or polymethacrylate) backbone generally have a very limited stability range with regard to operating temperatures, with temperatures above 120°C normally leading to irreversible destruction of the resin and loss of catalytic activity.

In US-A-2,282,469 to Frolich there is disclosed a process for preparing methyl tertiary butyl ether over a catalyst comprising Kieselguhr impregnated with phosphoric acid at a temperature of about 175°F to 350°F.

In US-A-4,822,921 there is disclosed a method for producing MTBE by reacting tertiary butyl alcohol and methanol in the presence of a catalyst comprising an inert support, such as titania, having a phosphoric acid impregnated thereon.

In US-A-4,827,048 there is disclosed a method for producing MTBE by reacting tertiary butyl alcohol and methanol in the presence of a catalyst comprising a heteropoly acid such as 12-tungstophosphoric acid or 12-molybdophosphoric acid on an inert support, such as titania.

US-A-4,918,244 (Nelson) describes a process for the preparation of MTBE by reaction of TBA and MeOH in a rectification tower having a packed solid-acid catalyst bed. A large number of catalyst types are disclosed, Amberlyst® 15 being exemplified.

EP-A-0,235,998 (Dow) describes supported fluorocarbonsulfonic acid polymers and methods for their preparation. Such polymers may be used as catalysts *per se* or, preferably, coated onto a carrier. There is a comprehensive review of applications for supported fluorocarbon sulfonic acid polymer heterogeneous catalysts by J.D. Weaver et al, Catalysis, 483 (1987).

It is an object of the present invention to selectively synthesize alkyl tertiary alkyl ether such as methyl tertiary butyl ether from tertiary alcohol, e.g. tertiary butyl alcohol and primary alcohol, e.g. methanol in one step using a catalyst which allows for rapid conversion of tertiary alcohol. It has now been discovered that fluorocarbon sulfonic acid polymers on inert supports can be used as catalysts for the selective synthesis of alkyl tertiary alkyl ether from such alcohols. The accompanying examples demonstrate good yields of MTBE using the fluorocarbon sulfonic acid polymers on silicon carbide and alumina.

In accordance with certain of its aspects, the present invention provides a method for preparing alkyl tertiary-alkyl ether from tertiary alkyl alcohol and C₁-C₆ primary alcohol in one-step which comprises reacting the tertiary alkyl alcohol and primary alcohol in the presence of a catalyst comprising a fluorocarbon sulfonic acid polymer on an inert support at an elevated temperature and moderature pressure. Most preferably the invention provides a method for preparing methyl tert-butyl ether from tertiary-butyl alcohol and methanol by said process. The following description concentrates upon the reaction of methanol and tert-butyl alcohol to prepare MTBE. This important reaction does not restrict the scope of this invention. Pefluorinated sulfonic acid resin polymers supported on inert carriers such as silicon carbide and alumina are particularly preferred.

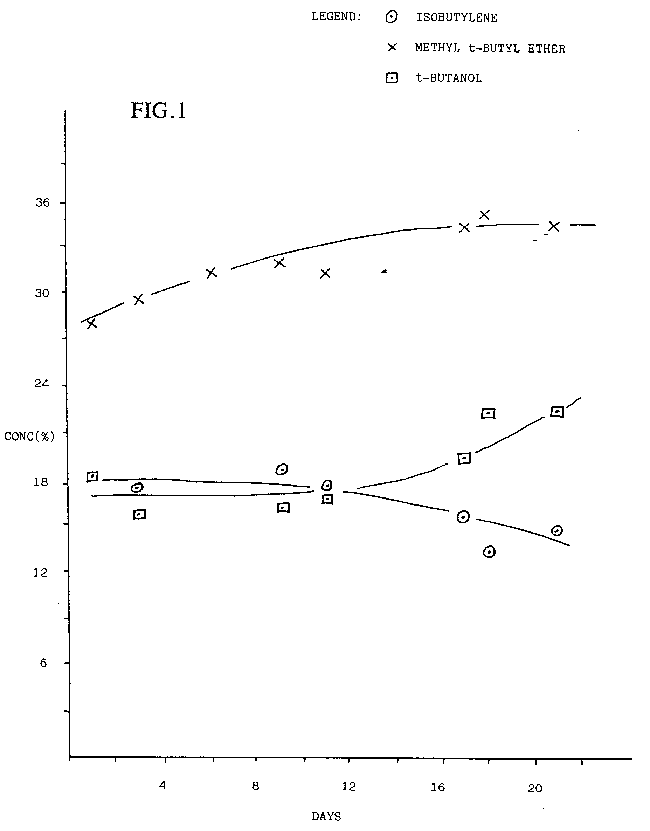

Fig. 1 shows the concentration of MTBE, isobutylene and t-butanol in the product over a 21 day period using the flurocarbonsulfonic acid polymer on silicon carbide.

The preparation of MTBE can be represented by the following:

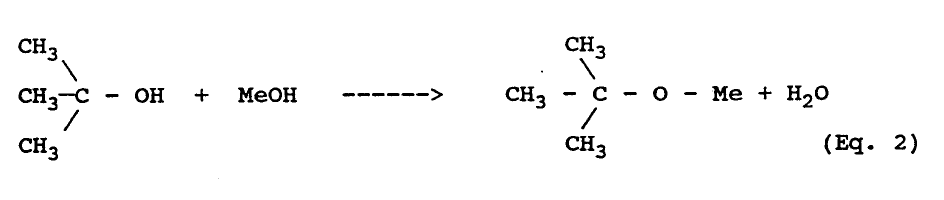

Generally the methanol and t-butanol coreactants may be mixed in any proportion in order to generate the desired methyl t-butyl ether, but preferably the molar ratio of methanol to t-butanol in the feed mixture should be between 10:1 and 1:10, if the yield of desired MTBE is to be maximized. In order to achieve maximum selectivity to MTBE, and optimum conversion per pass, an excess of methanol in the liquid feed is desirable. The most preferred methanol-to-tertiary butanol molar ratio is from 1:1 to 5:1.

In certain circumstances, it may be particularly desirable that the TBA conversion be high enough (e.g. >80% per pass), such that the crude product mix phase separates into an isobutylene-MTBE product-rich phase and a heavier aqueous methanol phase. Preferably such a product phase separation would be achieved at as low an etherification temperature as possible, but particularly in the range 160-200°C. This condition is illustrated in the accompanying Example 2.

The same process may also be applied to the preparation of other alkyl tertiary alkyl ethers. For example, said process may be applied to the reaction of a C₁-C₆ primary alcohol such as methanol, ethanol, n-propanol and n-hexanol with a C₄-C₁₀ tertiary alcohol such as, for example, tertiary butanol and tertiary amyl alcohol. The reaction of methanol with tertiary amyl alcohol (2-methyl-2-butanol) would then yield methyl tertiary amyl ether (TAME). Alternatively a mixture of alcohols, e.g., a mixture of C₁-C₅ alcohols, could be reacted to give a mixture of alkyl tert-alkyl ethers.

The fluorocarbon polymers are preferably perfluorinated sulfonic acid polymers represented by the structure: 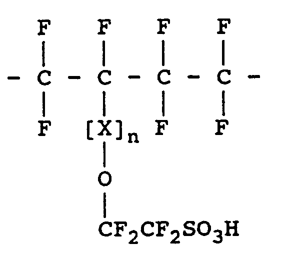 where
n = O or n = 1, 2, 3...;
X =

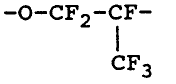

These are generally copolymers of tetrafluoroethylene and fluorinated vinyl ethers, which contain fluorosulfonyl groups. They contain repeating units of fluoroethylene with fluorocarbon side chains connected through ether linkages. Except under extreme conditions FSA polymers are essentially insoluble in most materials.

The sulfonic acid group of fluorocarbonsulfonic acid exhibits exceptionally high acid strength which results from the strong electron withdrawing effect of the fluorocarbon portion of the molecule. The Hammett Hₒ acidity function of the FSA polymer has been estimated to be -10 to -12, which makes it comparable to or stronger than 100% sulfuric acid. The titratable acidity of said polymers is typically 0.10 to 0.40 meq/g.

It is beneficial to increase the surface area of the catalyst and one method of increasing surface area without the inconvenience of handling fine powders is to support the polymer as a thin coating on a porous carrier.

The carrier on which the polymer is supported should be inert. Compounds which may be employed are those containing elements of Group III and IV of the Periodic Table. Suitable compounds include the oxides of aluminum, silicon, titanium and zirconium or combinations thereof, e.g. alumina, silica (silicon dioxide), titania (titanium dioxide) and zirconia, as well as combinations thereof. Also suitable are the various forms of carbon, other ion-exchange resins, and carbon-containing supports including certain carbides such as silicon carbide. Good results were observed using alumina and silicon carbide.

The catalyst of this invention preferably comprises fluorocarbon polymer dispersed on an alumina or silicon carbide support using a proprietary process.

The polymers can be dispersed on the carrier by a variety of methods. The preformed, inert carrier may, optionally, be calcined or dried prior to use as an etherification catalyst. Drying under vacuum, in air, or in an inert gas environment, such as nitrogen, may be conducted at a temperature of at least 100°C, but below the temperature at which thermal destruction leads to catalyst deactivation. This can be determined by routine experimentation for a particular catalyst. The catalyst is typically calcined or dried for from 1 to 24 hours at a temperature of from about 100° to 200°C.

An example of a commercial polymer containing these fluorinated compounds is XUS-40036.02 from DOW. XUS-40036.02 is a fluorosulfonic acid polymer on a silicon carbide carrier with a 36mm² (¼˝ x ¼˝) hollow cylinder having an acid capacity of 0.20 meq/g. XUS-40036.01, also manufactured by Dow, is a perfluorinated sulfonic acid polymer supported on an alumina carrier as 1.6mm (1/16˝) spheres having an acid capacity of 0.14 meq/g.

The weight percent of fluorocarbon sulfonic acid polymer to support should be such that the concentration of the sulfur in the formulated catalyst is in the range of 0.01 wt% to 10 wt%, although concentrations outside this range may also be employed. Where fluorocarbon sulfonic acid, for example, is supported on silicon carbide, a suitable quantity of sulfur is >0.01 wt%.

The inert support may be in the form of powders spheres, tablets, extrudates or cylinders. It will be understood that pellets of any suitable shape and dimensions may be used as desired by one wishing to practice the process of the present invention. Cylindrically-shaped catalyst pellets having a diameter essentially equal to the length thereof can be employed with good results. Diameters ranging from about 0.794 mm (1/32 inch) to about 9.525 mm (3/8 inch) possess desirable dimensions.

As will be demonstrated by the examples, the supports are preferably of high purity and high surface area. It has been found in the process of this invention that greater conversion of tertiary butanol and methanol is achieved where the surface area of the support is generally >10 m²/g.

When cylindrical pellets of catalyst of the type described above are used, the liquid hourly space velocity may be varied within wide limits (e.g., 0.1 to 10) in order to obtain a desired rate of conversion, as explained above. Normally, space velocities of about 0.1 to 10 LHSV will be employed. Excellent t-butanol conversion levels are observed under forcing conditions, with essentially no loss in activity over extended periods.

The catalyst compositions of the present invention are preferably employed as a fixed bed of catalyst in a continuous reaction system. In a continuous process of this nature, the time of contact of the reactants with the catalyst is one of the interrelated factors that those skilled in the art will adjust, along with temperature, pressure, bed geometry, pellet size, etc. in order to obtain a desired rate of reaction and, hence, a desired percentage of conversion of the reactants. Thus, in a continuous process, it is not necessary to drive the reaction to completion because unreacted feedstock components can be recycled to the reactor.

Catalyst life is an important factor in conducting a continuous reaction. For example, if a catalyst is easily poisoned, or if catalyst pellets do not have good structural properties, the economics of the process will be adversely affected. The catalysts of the present invention possess such desirable physical properties as high acid strength and stability at elevated temperatures and are relatively less sensitive to poisoning, so this should not present a problem.

As a consequence, the catalyst compositions of the present invention are advantageously used in a continuous process for the continuous production of methyl t-butyl ether reaction products from tertiary butanol and methanol. Such catalyst compositions can be used for prolonged periods without the need for regeneration. Nevertheless, with the passage of time deactivation will tend to slowly occur. Deactivation can be measured qualitatively by the loss of butanol conversion, or as the increase of temperature required to maintain an essentially constant conversion rate for the t-butanol and methanol.

The fact that this method can be achieved under relatively mild operating conditions is an attractive feature of this invention. Etherification can generally be conducted at temperatures from 20° to 250°C. The preferred range is 100° to 200°C. The total operating pressure may be from 0 to 6895 kPa gauge pressure (gp) (0 to 1000 psig). The preferred pressure range is 344 to 3440 kPa gp (50 to 500 psig).

The reaction may be carried out in either a stirred slurry reactor or in a fixed bed continuous flow reactor. The catalyst concentration should be sufficient to provide the desired catalytic effect.

Typically, MTBE is generated continuously in up to ca. 45 wt% concentration in the crude liquid product at total liquid hourly space velocities (LHSV) of at least 0.1 and up to ten, and relatively mild conditions, where:$\text{LHSV =} \frac{\text{Volume Of Total Liquid Feed Run Through The Reactor Per Hour}}{\text{Volume of Catalyst In Reactor}}$

The examples which follow illustrate the one-step synthesis of MTBE from TBA and MeOH (Eq. 2) using fluorocarbon sulfonic acid polymers particularly supported on hollow cylinders or spheres. The examples are only intended as a means of illustration and it is understood the invention is not meant to be limited thereby.

Conversion of t-butanol (TBA, wt%) is estimated in the following examples using the equation:$\frac{\text{(Wt% Conc. of TBA in Feed - Wt% Conc. of TBA in Product)}}{\text{Wt% Conc of TBA in Feed}} \text{x 100}$

Selectivities to methyl t-butyl ether (MTBE, mole %) and isobutylene (C₄H₈ mol%) are estimated from:$\frac{{\text{Moles of MTBE (or C}}_{\text{4}} {\text{H}}_{\text{8}} \text{) in Product Liquid}}{\text{Moles of TBA Converted}} \text{x 100}$

### EXAMPLE 1

This example illustrates the production of methyl t-butyl ether from t-butanol and methanol using a perfluorinated sulfonic acid polymer supported on a silicon carbide carrier. Synthesis was conducted in a tubular reactor 14mm x 300mm (0.56˝ diameter, 12˝ long), constructed of 316 stainless steel, operated upflow and mounted in a furnace, controllable to ±1.0°C and fitted with pumps allowing flow control to <±1cc/hr. The reactor was also fitted with a pressure regulating device and equipment for monitoring temperature, pressure and flow rate.

The reactor was charged at the beginning of the experiment with 25cc of perfluorinated sulfonic acid polymer supported on a silicon carbide carrier (Sample XUS-40036.02 from Dow) as 6mm x 6mm (¼˝ x ¼˝) hollow cylinders having an acid capacity of 0.20 meq/g. A screen of glass wool was placed at the top and bottom of the reactor to ensure the catalyst would remain in the middle portion.

The catalyst bed was treated with a methanol/t-butanol (1.1:1 molar mix) upflow, at a flow rate of 50cc/hr, while the reactor was held at 160°C, with a total pressure of 2.16 MPa (300 psig). Samples of crude product effluent were collected periodically on-stream, in 316ss bombs, and analyzed by GLC and GC-IR.

Typical analysis data for samples taken under these conditions are summarized in Table 1. Concentrations of isobutylene, MTBE and t-butanol in these crude effluent products are also plotted in Fig. 1. For Samples 2 and 8, taken after 3 and 21 days operating time, respectively, the t-butanol conversion levels and MTBE and isobutylene selectivities are as follows:

| | **Sample #2 (3 Days)** | **Sample #8 (21 Days)** |
|---|---|---|
| t-Butanol Conversion (%) | 78 | 70 |
| Isobutylene Selectivity (Mole%) | 42 | 39 |
| MTBE Selectivity (Mole%) | 46 | 59 |

### EXAMPLE 2

This example illustrates the production of methyl t-butyl ether from t-butanol and methanol using a perfluorinated sulfonic acid polymer supported on an alumina carrier.

Synthesis was conducted using the equipment and procedures of Example 1. The reactor was charged with 25cc of perfluorinated sulfonic acid polymer supported on an alumina carrier (Sample XUS-40036.01 from Dow) as 1.6mm (1/16") spheres having a acid capacity of 0.14 meq/g. The catalyst bed was then treated with a methanol/t-butanol (1.1:1 molar mix) at a flow rate of 50cc/hr while the temperature was 160°C and the total pressure 2.06 MPa (300 psi). Samples of crude product were collected periodically on-stream, in 316ss bombs and analyzed by GLC.

Concentrations of isobutylene, MTBE and t-butanol in the crude two-phase effluent products are summarized in Table 2 for a 24 day run. For Samples 1 and 17 taken after 1 and 24 days, respectively, the t-butanol conversion levels and MTBE and isobutylene selectivities are as follows:

| | **Sample #1 (1 Day)** | **Sample #17 (24 Days)** |
|---|---|---|
| t-Butanol Conversion (%) | 88 | 86 |
| Isobutylene Selectivity (Mole%) | 58 | 51 |
| MTBE Selectivity (Mole%) | 44 | 51 |

### EXAMPLE 3

This example illustrates the production of methyl t-butyl ether from t-butanol and methanol using perfluorinated sulfonic acid polymer on a carrier over a range of operating conditions.

Synthesis was conducted using the equipment and procedures of Example 1. The reactor was charged with 25cc of perfluorinated sulfonic acid polymer supported on alumina (Sample XUS-40036.0l), and the catalyst bed was then treated with a methanol/t-butanol (1.1:1 molar mix) at a series of temperatures and flow rates, while maintaining pressure at 2.16 MPa (300 psig). Samples of crude product were collected in duplicate at each condition in 316ss bombs and analyzed by GLC.

Typical analyses data for samples were taken over a range of conditions are summarized in Table 3. For Samples 12 and 15 taken at 160°C and liquid space velocities of 2 and 5, respectively, the t-butanol conversion levels and MTBE and isobutylene selectivities are as follows:

| | **Sample #12 (LHSV = 2)** | **Sample #15 (LHSV = 5)** |
|---|---|---|
| t-Butanol Conversion (%) | 79 | 73 |
| Isobutylene Selectivity (Mole%) | 55 | 49 |
| MTBE Selectivity (Mole%) | 46 | 47 |

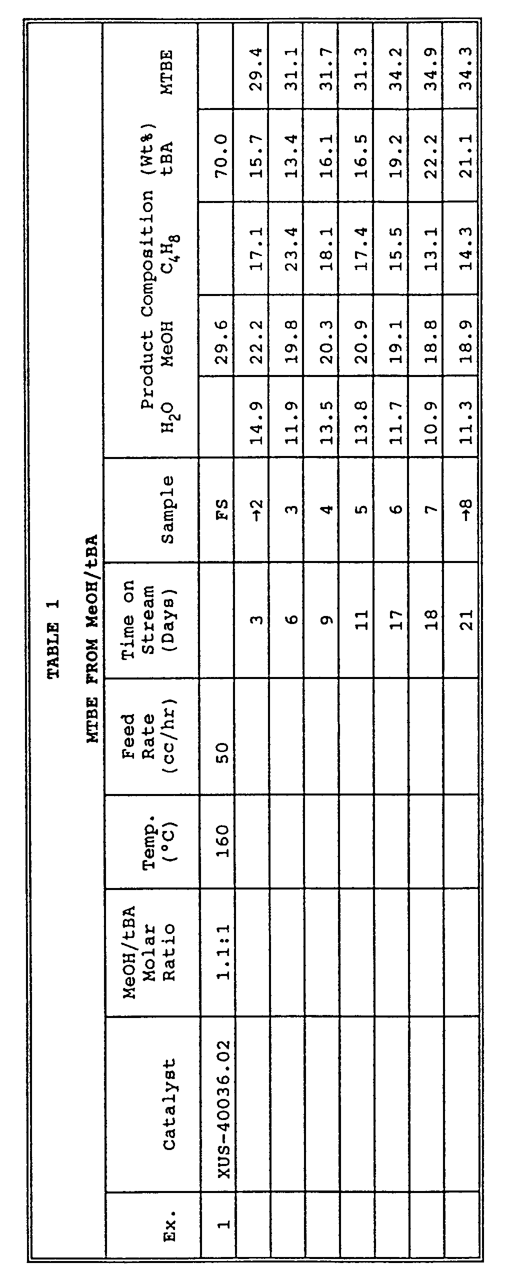

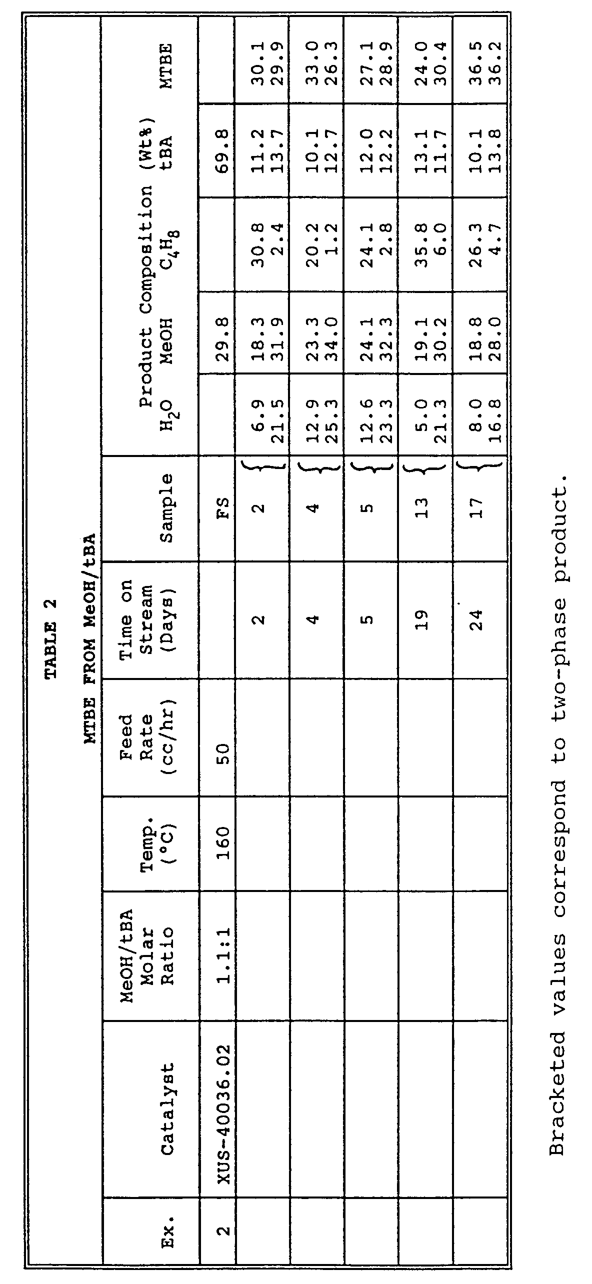

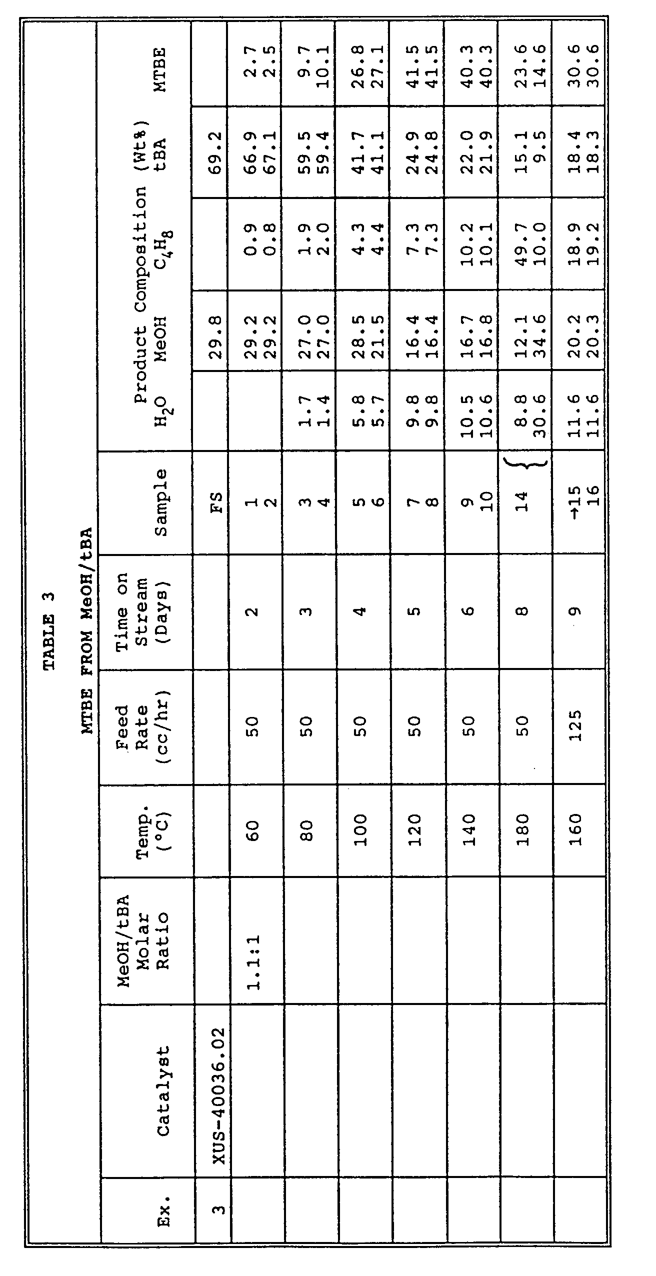

## Claims

1. A process for the preparation of alkyl tertiary alkyl-ether from tertiary alcohol and primary alcohol in the presence of a catalyst, characterised in that perfluorinated sulfonic acid polymer supported on an inert carrier is used as a catalyst.

2. A process according to Claim 1 for the preparation of methyl tert-butyl ether from t-butanol and methanol.

3. A process as claimed in Claim 2, wherein said t-butanol and methanol are present in a molar amount of 0.1 to 10 moles of methanol per mole of t-butanol.

4. A process as claimed in Claim 2 or Claim 3 wherein said t-butanol and methanol are continuously contacted with said catalyst at a temperature of 20°C to 250°C and a pressure of atmospheric to 6895 kPa gauge pressure (1000 psig) to obtain methyl tert-butyl ether.

5. A process as claimed in any one of Claims 1 to 3 wherein the perfluorinated sulfonic acid polymer is represented by the structure: 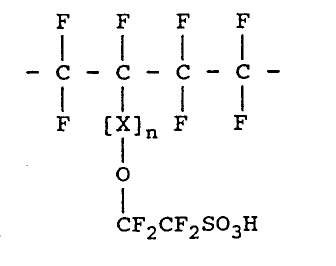 where
n = O or an integer and x has the structure:
X =

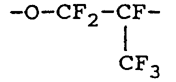

6. A process as claimed in any one of the preceding claims wherein the inert support contains an element from Group III or IV of the Periodic Table.

7. A process as claimed in Claim 6 wherein said inert support is selected from the group consisting of alumina, silicon dioxide or silicon carbide.

8. A process as claimed in any one of the preceding claims wherein the inert support has a cylindrical shape wherein the diameter is essentially equal to the length thereof.

9. A process as claimed in any one of Claims 2 to 8 wherein the operating temperature is in the range of 160° to 200°C and the product comprises a two-phase mix of an isobutylene-MTBE product-rich phase and a heavier aqueous-methanol rich phase.

10. The use of a perfluorinated sulfonic acid polymer supported on an inert carrier in a method of reacting t-butanol with methanol according to Claim 2.

## Patentansprüche

1. Ein Verfahren zur Herstellung von Alkyl-tert.-alkylether aus tertiärem Alkohol und primärem Alkohol in der Gegenwart eines Katalysators, dadurch gekennzeichnet, daß perfluoriertes Sulfonsäurepolymer, aufgebracht auf einem inerten Träger, als ein Katalysator verwendet wird.

2. Ein Verfahren nach Anspruch 1 zur Herstellung von Methyl-tert.-butylether aus tert.-Butanol und Methanol.

3. Ein Verfahren nach Anspruch 2, wobei besagtes tert.-Butanol und Methanol in einer molaren Menge von 0,1 bis 10 Mol Methanol pro Mol tert.-Butanol vorhanden sind.

4. Ein Verfahren nach Anspruch 2 oder Anspruch 3, wobei besagtes tert.-Butanol und Methanol kontinuierlich mit besagtem Katalysator bei einer Temperatur von 20°C bis 250°C und einem Druck von atmosphärisch bis 6.895 kPa Überdruck (1.000 psig) in Kontakt gebracht werden, um Methyl-tert.-butylether zu erhalten.

5. Ein Verfahren nach einem der Ansprüche 1 bis 3, wobei das perfluorierte Sulfonsäurepolymer dargestellt wird durch die Struktur: 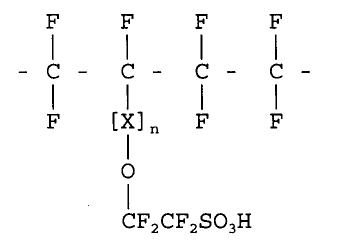 wobei
n = O oder eine ganze Zahl und X die Struktur besitzt:
X =

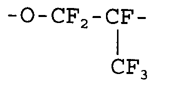

6. Ein Verfahren nach einem der vorangehenden Ansprüche, wobei der inerte Träger ein Element aus Gruppe III oder IV des Periodensystems enthält.

7. Ein Verfahren nach Anspruch 6, wobei besagter inerter Träger ausgewählt ist aus der Gruppe, die aus Aluminiumoxid, Siliciumdioxid oder Siliciumcarbid besteht.

8. Ein Verfahren nach einem der vorangehenden Ansprüche, wobei der inerte Träger eine zylindrische Form besitzt, wobei der Durchmesser im wesentlichen seiner Länge entspricht.

9. Ein Verfahren nach einem der Ansprüche 2 bis 8, wobei die Betriebstemperatur im Bereich von 160° bis 200°C liegt und das Produkt ein Zwei-Phasen-Gemisch aus einer an Isobutylen-MTBE-Produkt reichen Phase und einer schwereren wässrigen, an Methanol reichen Phase umfaßt.

10. Die Verwendung eines perfluorierten Sulfonsäurepolymers, aufgebracht auf einen inerten Träger, in einem Verfahren zum Umsetzen von tert.-Butanol mit Methanol nach Anspruch 2.

## Revendications

1. Procédé de préparation d'un éther alkylique tertio-alkylique à partir d'un alcool tertiaire et d'un alcool primaire en présence d'un catalyseur, caractérisé en ce qu'on utilise comme catalyseur un polymère d'acide sulfonique perfluoré sur un support inerte.

2. Procédé selon la revendication 1 pour la préparation d'un éther méthylique tertio-butylique à partir de tertio-butanol et de méthanol.

3. Procédé selon la revendication 2, dans lequel ce tertio-butanol et ce méthanol sont présents dans un rapport molaire de 0,1 à 10 moles de méthanol par mole de tertio-butanol.

4. Procédé selon la revendication 2 ou la revendication 3, dans lequel ce tertio-butanol et ce méthanol sont mis en contact en continu avec ce catalyseur à une température de 20°C à 250°C et sous une pression allant de la pression atmosphérique à 6895 kPa au manomètre (1000 psig) pour obtenir l'éther méthylique tertio-butylique.

5. Procédé selon l'une quelconque des revendications 1 à 3, dans lequel le polymère d'acide sulfonique perfluoré répond à la formule : 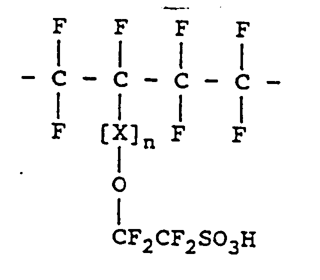 dans laquelle n = 0 ou un entier et X répond à la formule :
X =

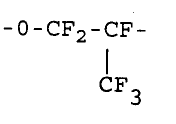

6. Procédé selon l'une quelconque des revendications précédentes, dans lequel le support inerte contient un élément du Groupe III ou IV de la Classification Périodique.

7. Procédé selon la revendication 6, dans lequel ce support inerte est choisi dans le groupe constitué de l'alumine, du dioxyde de silicium ou du carbure de silicium.

8. Procédé tel que revendiqué dans l'une quelconque des revendications précédentes, dans lequel le support inerte a une forme cylindrique dans laquelle le diamètre est pratiquement égal à la longueur.

9. Procédé tel que revendiqué dans l'une quelconque des revendications 2 à 8, dans lequel la température opératoire est dans l'intervalle de 160° à 200°C et le produit comprend un mélange à deux phases d'une phase riche en produit isobutylène-MTBE et une phase riche en méthanol aqueux plus lourde.

10. Utilisation d'un polymère d'acide sulfonique perfluoré sur un support inerte dans un procédé consistant à faire réagir le tertio-butanol avec le méthanol conformément à la revendication 2.
